(19) Europäisches Patentamt
European Patent Office
Office européen des brevets

(11) Veröffentlichungsnummer : **0 091 052**
**B1**

(12) # EUROPÄISCHE PATENTSCHRIFT

(45) Veröffentlichungstag der Patentschrift : **03.12.86**

(21) Anmeldenummer : **83103044.0**

(22) Anmeldetag : **28.03.83**

(51) Int. Cl.⁴ : **C 07 C147/14**, C 07 F 9/165, C 07 D333/48

(54) **Industrielles Verfahren zum Oxydieren von Thioethern und Thiolcarbonsäure-Derivaten.**

(30) Priorität : **06.04.82 JP 56081/82**

(43) Veröffentlichungstag der Anmeldung : **12.10.83 Patentblatt 83/41**

(45) Bekanntmachung des Hinweises auf die Patenterteilung : **03.12.86 Patentblatt 86/49**

(84) Benannte Vertragsstaaten : **BE CH DE FR GB IT LI**

(56) Entgegenhaltungen :
**DE-A- 2 413 574**
**DE-A- 2 450 634**
**DE-B- 1 290 008**

(73) Patentinhaber : **NIHON TOKUSHU NOYAKU SEIZO K.K.**
**No.4, 2-Chome, Nihonbashi Honcho**
**Chuo-ku Tokyo 103 (JP)**

(72) Erfinder : **Saito, Junichi**
**3-7-12, Osawa**
**Mitaka-shi Tokyo (JP)**
Erfinder : **Shiokawa, Kozo**
**210-6, Shukugawara Tama-ku**
**Kawasaki-shi Kanagawa-ken (JP)**
Erfinder : **Takemoto, Toshiyuki**
**50-3-206, Matsugaya**
**Hachioji-shi Tokyo (JP)**

(74) Vertreter : **Schumacher, Günter, Dr. et al**
**c/o Bayer AG Konzernverwaltung RP Patentabteilung**
**D-5090 Leverkusen 1 Bayerwerk (DE)**

**EP 0 091 052 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein verbessertes Verfahren, durch das Sulfoxid-Verbindungen industriell vorteilhaft aus Thioethern oder Thiolcarbonsäure-Derivaten hergestellt werden können ; das Verfahren liefert die Sulfoxid-Verbindungen mit deutlich höherer Reinheit und in ausgeprägt höheren Ausbeuten und bietet Vorteile sowohl in bezug auf die betriebliche Durchführung als auch in bezug auf die Produktionskosten, wobei eine hohe Sicherheit gewährleistet ist und die verschiedenartigen Nachteile und Schwierigkeiten einer praktischen Arbeitsweise im industriellen Maßstab vermieden werden.

Insbesondere betrifft die vorliegende Erfindung ein Verfahren zur Herstellung eines Sulfoxids der nachstehenden Formel

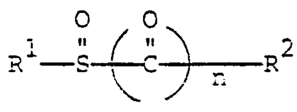

(1)

in der $R^1$ und $R^2$ gleich oder verschieden sind und jeweils für eine Alkyl-, Alkenyl- oder Alkinyl-Gruppe, die jeweils durch Halogen, Alkoxy, einen Phosphorsäureester-Rest, eine Phenyl-Gruppe oder im Falle von Alkyl eine durch 1 bis 5 Halogenatome oder 1 bis 5 Cyano-, Nitro-, Alkyl-, Alkoxy- oder Phenyloxy-Gruppen substituierte Phenyl-Gruppe substituiert sein können, eine Aryl-Gruppe, die durch ein Halogen-Atom oder eine Nitro-, Alkyl-, Alkoxy-, Cyano-, Alkenyl-, Alkinyl-, Aryloxy-, Alkylcarbonyl- oder Alkoxycarbonyl-Gruppe substituierte sein kann, oder eine Gruppe der Formel

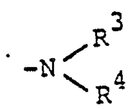

stehen, in der

$R^3$ und $R^4$ gleich oder verschieden sind und jeweils für ein Wasserstoff-Atom oder eine Alkyl-, Alkenyl-, Alkinyl- oder Phenyl-Gruppe stehen oder $R^3$ und $R^4$ miteinander verbunden sein können und einen $C_3$- bis $C_6$-Ring bilden, der gegebenenfalls ein anderes Hetero-Atom enthält, und

n 0 oder 1 ist, und wenn n 0 ist, $R^1$ und $R^2$ miteinander verbunden sein können, so daß sie einen Ring mit $C_4$ bis $C_6$ bilden,

das die Reaktion eines Thioethers oder eines Thiolcarbonsäure-Derivats der Formel

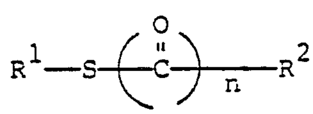

(2)

in der $R^1$, $R^2$ und n die im Vorstehenden angegebenen Bedeutungen haben, zur Oxidation desselben mit Wasserstoffperoxid umfaßt und das

dadurch gekennzeichnet ist, daß die Reaktion in einem aprotischen Lösungsmittel in Gegenwart einer organischen Carbonsäure unter durch Schwefelsäure herbeigeführten sauren Bedingungen durchgeführt wird.

Es ist bekannt, daß Salpetersäure, Chromsäure, Permangansäure, Peroxybenzoesäure etc. im allgemeinen als Oxidationsmittels bei der Oxidation von Thiothern zu Sulfoxiden verwendet werden. Wenn beispielsweise Salpetersäure als Oxidationsmittel bei der Oxidation einer Thioether-Verbindung mit einer Phenyl-Gruppe an einer Seite verwendet wird, kann manchmal gleichzeitig mit der angestrebten Oxidationsreaktion als Nebenreaktion die Nitrierung am Phenyl-Kern stattfinden. Weiterhin ist es so, daß bei Verwendung von Chromsäure und von Permangansäure als Oxidationsmittel die Oxidation weiter zu den Sulfonen fortschreitet und es schwierig ist, die angestrebten Sulfoxide selektive zu gewinnen. Peroxybenzoesäure ist teuer, und Reaktionen, bei denen sie eingesetzt wird, schaffen, wie in den Fällen der anderen, im Vorstehenden erwähnten Oxidationsmittel Probleme hinsichtlich der Gefährlichkeit und der Wasserverschmutzung. Dementsprechend machen es diese verschiedenen Mängel und Unannehmlichkeiten bei der praktischen Durchführung im industriellen Maßstab schwierig, das angestrebte Produkt mit hoher Reinheit und in hoher Ausbeute günstig mittels einer Arbeitsweise herzustellen, die für die industrielle Praxis geeignet ist.

Als ein anderes Oxidationsverfahren zeigt die JP-Patentveröffentlichung Nr. 778/1960 ein Beispiel einer Reaktion unter Verwendung von Wasserstoffperoxid in einem Essigsäure-Lösungsmittel auf. Es wurde jedoch gefunden, daß bei der Durchführung der Reaktion nach dieser Arbeitsweise in der industriellen Praxis verschiedene Schwierigkeiten bei der Abtrennung des angestrebten Produkts aus der

Reaktionsmischung auftreten. Während beispielsweise bei einem Versuch im Laboratoriumsmaßstab, der in den oben bezeichneten Patentunterlagen beschrieben ist, das gewünschte Produkt dadurch extrahiert wird, daß die Reaktionsmischung mehrmals zusammen mit einem organischen Lösungsmittel und Wasser geschüttelt wird, ist es bei der Durchführung dieses Reaktionsverfahrens im industriellen Maßstab erforderlich, die Reaktionsmischung in eine große Menge Wasser hineinzugießen, damit eine Mischung aus der Essigsäure und der großen Menge Wasser gebildet wird, und dann diese Mischung mit einem organischen Lösungsmittel zu extrahieren, um das angestrebte Produkt aus ihr abzutrennen und zu isolieren. Die Menge des für die Gewinnung des gewünschten Produkts zu behandelnden Materials wird gewaltig, und für die Beseitigung einer großen Menge des die Essigsäure enthaltenden Abwassers muß gesorgt werden. Darüber hinaus ist der Arbeitsgang der Rückgewinnung der Essigsäure aus der sie enthaltenden wäßrigen Lösung in der industriellen Praxis sehr unvorteilhaft und schwierig, und Behandlungsanlagen hoher Kapazität werden für die Behandlung einer großer Menge des die Essigsäure enthaltenden Abwassers benötigt. Infolgedessen wirft die im Vorstehenden beschriebene Arbeitsweise viele technische Probleme auf, die sie für die Industriepraxis ungeeignet machen. Andererseits gibt es ein Verfahren, bei dem die Reaktionsmischung, die Wasserstoffperoxid und das Reaktionsprodukt in dem Essigsäure-Lösungsmittel enthält, unmittelbar einer Destillation unterworfen wird, um die Essigsäure abzudestillieren, wie dies im Laboratoriumsmaßstab in den oben genannten Patentunterlagen geschieht. Bei diesem Verfahren ist es jedoch schwierig, einen sicheren Betriebsablauf zu gewährleisten, und diese Arbeitsweise birgt Explosionsgefahr in sich. Infolgedessen ist es schwierig, dieses Verfahren in der industriellen Praxis einzusetzen.

Die JP-Patentveröffentlichung Nr. 778/1960 zeigt auch ein Beispiel einer Reaktion unter Verwendung von Wasserstoffperoxid in einem Methanol-Lösungsmittel unter Bedingungen auf, bei denen mit Schwefelsäure angesäuert wurde. Es wurde gefunden, daß auch bei der Arbeitsweise diese Reaktion die Abtrennung des angestrebten Produkts aus der Reaktionsmischung in der industriellen Praxis viele Schwierigkeiten bereitet, und während des Ablaufs der Reaktion treten verschiedenartige Schwierigkeiten auf. Bei der praktischen Durchführung dieser Reaktion im industriellen Maßstab ergeben sich die gleichen Probleme, wie sie im Vorstehenden dargelegt wurden, in bezug auf die Behandlung des methanolhaltigen Abwassers, und ein komplexer und unvorteilhafter Arbeitsschritt wird für die Rückgewinnung und Wiederverwendung des Methanols erforderlich. Überdies erfordert dieses Verfahren den Einsatz einer kostspieligen Destillationsvorrichtung. Die betreffenden Patentunterlagen geben an, daß bei diesem Reaktionsvorgang die Reaktionstemperatur 40 °C bis 50 °C beträgt. Als Ergebnis der Nacharbeitung dieses Reaktionsverfahrens wurde seitens der Anmelderin gefunden, daß die Reaktion nur langsam abläuft und daß zur Erzielung einer vollständigen Umsetzung im industriellen Maßstab das Erhitzen auf 40 °C bis 50 °C, häufig sogar auf etwa 60 °C, über einen beträchtlich langen Zeitraum hinweg durchgeführt werden muß. Es wurde ebenfalls gefunden, daß die Gefahr besteht, daß eine anomale Reaktion ausgelöst wird, und dieses Reaktionsverfahren ist für den praktischen Einsatz in der Industrie ebenfalls nicht geeignet.

Die Anmelderin hat Untersuchungen zur Entwicklung eines industriellen Verfahrens durchgeführt, das zur Herstellung einer Sulfoxid-Verbindung im industriellen Maßstab geeignet ist und bei dem in zweckmäßiger und selektiver Weise ein Thioether oder ein Thiolcarbonsäure-Derivat oxydiert wird, ohne daß je nach den Eigenschaften der Ausgangs-Verbindung der Einsatz verschiedenartiger Oxidationsmittel erforderlich wird.

Während gemäß dem Stand der Technik die Reaktion mit Wasserstoffperoxid in einem Methanol-Lösungsmittel mit hoher Polarität unter sauren Bedingungen, die durch eine kleine Menge Schwefelsäure hergestellt wurden, oder mit Wasserstoffperoxid in einem Essigsäure-Lösungsmittel mit hoher Polarität durchgeführt wird und die Ausgangs-Verbindung vermittels des Oxidationsvermögens der entstehenden organischen Persäure in das angestrebte Endprodukt umgewandelt wird, wurde nunmehr seitens der Anmelderin aufgrund der genannten Untersuchungen gefunden, daß die oben erwähnten Mängel und Nachteile der Verfahrens gemäß dem Stand der Technik sich in vorteilhafter Weise dadurch vermeiden lassen, daß die genannte Reaktions in einem aprotischen Lösungsmittel in Gegenwart einer beträchtlich verminderten Menge, die beispielsweise kleiner ist als das Äquivalent der Ausgangs-Verbindung, einer organischen Carbonsäure unter sauren Bedingunden, die durch eine kleine Menge Schwefelsäure herbeigeführt wurden, durchgeführt werden kann und daß dadurch ein Verfahren zur Herstellung einer Sulfoxid-Verbindung im industriellen Maßstab verfügbar gemacht werden kann, das verschiedene Vorteile bietet, etwa die in ausgeprägter Weise erhöhten Reinheitsgrade und Ausbeuten, betriebliche und wirtschaftliche Vorteile, gute Reproduzierbarkeit der Qualität des Produkts, die beträchtliche Verminderung der Menge der organischen Carbonsäure, niedrige Reaktionstemperaturen, abgekürzte Reaktionszeiten sowie verschiedene Verbesserungen in bezug auf die Entsorgung des Abwassers.

Dementsprechend ist ein Ziel der vorliegenden Erfindung, ein verbessertes Verfahren verfügbar zu machen, bei dem die selektive Oxidation eines Thioethers oder eines Thiolcarbonsäure-Derivats zu einer Sulfoxid-Verbindung industriell in vorteilhafter Weise durchgeführt werden kann.

Der Ablauf der Reaktion nach dem Verfahren gemäß der vorliegenden Erfindung kann schematisch wie folgt dargestellt werden :

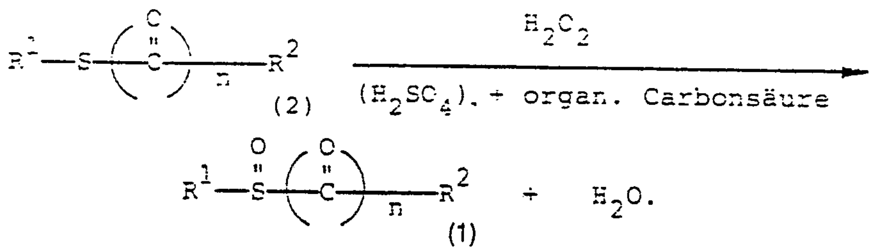


(2)

(1)

In dem vorstehenden Schema haben $R^1$, $R^2$ und n die im Vorstehenden angegebenen Bedeutungen.

Spezielle Beispiele für $R^1$ und $R^2$ sind : $C_1$- bis $C_8$-, vorzugsweise $C_1$- bis $C_6$-Alkyl-Gruppen wie Methyl, Ethyl, n- (oder iso-), Propyl, n- (iso-, sec- oder tert-)-Butyl, n- (oder iso-) Amyl und n-Hexyl ; Halogenoalkyl-Gruppen, die $C_1$- bis $C_8$-, vorzugsweise $C_1$- bis $C_6$-Alkyl-Gruppen wie oben beispielhaft bezeichnet enthalten, wie chloro-, bromo-, fluoro- oder iodo-substituierte Alkyl-Gruppen ; $C_1$- bis $C_8$-, vorzugsweise $C_1$- bis $C_6$-Alkyl-Gruppen wie oben beispielhaft bezeichnet, die $C_1$- bis $C_8$-, vorzugsweise $C_1$- bis $C_6$-Alkoxy-Gruppen enthalten, wie methoxy-, ethoxy-, n- (oder iso-) propoxy-, n- (iso-, sec- oder tert-) butoxy, n- (oder iso-)-amyloxy- oder n-hexyloxy-substituierte Alkyl-Gruppen ; O,O-Dialkyl-, oder O,S-Dialkylphospho (oder -thiophospho)-S- (oder -O-) alkyl-Gruppen, in denen die Alkyl-Struktureinheiten $C_1$- bis $C_8$-, vorzugsweise $C_1$- bis $C_6$-Alkyl-Gruppen wie oben beispielhaft bezeichnet sind ; phenylsubstituierte Alkyl-Gruppen, in denen die Phenyl-Struktureinheit durch 1 bis 5 Halogen-Atome oder 1 bis 5 Cyano-, Nitro-, Alkyl-, Alkoxy- oder Phenyloxy-Gruppen substituiert sein können ; $C_2$- bis $C_6$-, vorzugsweise $C_2$- bis $C_4$-Alkenyl-Gruppen wie Vinyl, Allyl oder Methallyl ; $C_2$- bis $C_6$-, vorzugsweise $C_2$- bis $C_4$-Alkinyl-Gruppen wie Ethinyl, Propinyl oder Butinyl ; $C_6$- und $C_{10}$-Aryl-Gruppen, die 1 bis 5 Substituenten haben können, wie etwa eine Phenyl- oder Naphthyl-Gruppe, die durch mindestens einen Substituenten ausgewählt aus der Klasse bestehend aus $C_1$- bis $C_8$-Alkyl-Gruppen, Halogen-Atomen, wie sie oben in bezug auf die Halogenoalkyl-Gruppen beispielhaft bezeichnet sind, $C_1$- bis $C_8$-Alkoxy-Gruppen, Cyano-Gruppen, Nitro-Gruppen, $C_2$- bis $C_6$-Alkenyl-Gruppen, $C_2$- bis $C_6$-Alkinyl-Gruppen, Phenyloxy-Gruppen, die gegebenenfalls einen Substituenten tragen, $C_1$- bis $C_8$-Alkylcarbonyl-Gruppen und $C_1$- bis $C_8$-Alkoxy-carbonyl-Gruppen substituiert sein kann ; Amino-Gruppen wie die N-Alkylamino-, N,N-Dialkylamino-, N-Alkenylamino-, N,N-Dialkenylamino-, N-Alkinylamino-, N,N-Dialkinylamino-, N-Phenylamino- und N-Alkyl-N-phenylamino-Gruppe und $C_4$- bis $C_7$-Ringe, die durch Bindung von $R^3$ und $R^4$ aneinander gebildet sind, wie die Gruppe 1-Pyrrolidino-, Piperidino-, Hexamethylenimino-, Heptamethylenimino- und Morpholino ; sowie Tetrahydrothiophen oder Tetrahydrothiopyran, entsprechend dam Fall, in dem n 0 ist und $R^1$ und $R^2$ aneinander gebunden sind, so daß sie einen Ring mit 4 bis 6 Kohlenstoff-Atomen bilden.

Als Beispiele für die Ausgangs-Verbindungen der Formel (2) können Dialkylthioether, Alkylarylthioether, Diarylthioether, Thiolcarbonsäuren, Thiolcarbonsäureester, Thiocarbamate und heterocyclische Verbindungen mit einem Schwefel-Atom im weitesten Sinne erwähnt werden, denen auf dem Gebiet der Chemie Bedeutung zukommt. Wenn das Verfahren gemäß der vorliegenden Erfindung auf diese Ausgangsstoffe angewandt wird, ist es als industrielles Verfahren zur selektiven Oxidation den Verfahren, die sich anderer Oxidationsmittel bedienen, und ähnlichen Verfahren beträchtlich überlegen.

Nach dem Verfahren gemäß der vorliegenden Erfindung wird die Reaktion der Verbindung der Formel (2) mit Wasserstoffperoxid zur Erzeugung der Verbindung der Formel (1) in Gegenwart einer organischen Carbonsäure in einem aprotischen Lösungsmittel unter durch Schwefelsäure-Zusatz hergestellten sauren Bedingungen durchgeführt.

Die Reaktion wird in einem im wesentlich mit Wasser nicht mischbaren oder in Wasser unlöslichen aprotischen Lösungsmittel durchgeführt, vorzugsweise mindestens einem aprotischen Lösungsmittel ausgewählt aus der aus Kohlenwasserstoff-Lösungsmitteln, halogenierten Kohlenwasserstoff-Lösungsmitteln, Ether-Lösungsmitteln und Keton-Lösungsmitteln bestehenden Gruppe. Zu Beispielen für solche aprotischen organischen Lösungsmittel zählen aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol ; Ether wie Diethylether, Methylethylether, Di-isopropylether und Dibutylether ; sowie Ketone wie Methylisobutylketon. Diese können entweder einzeln oder in Form von Gemischen eingesetzt werden.

Beispiele für bevorzugte organische Carbonsäure, die in der oben bezeichneten Reaktion verwendet werden, sind Ameisensäure, Essigsäure, Propionsäure, Halogen-Substitutionsprodukte dieser Säuren und Chlorobenzoesäuren. Spezielle Beispiele für die Halogen-Substitutionsprodukte sind Monochloroessigsäure, Dichloroessigsäure und Trichloroessigsäure. m-Chlorobenzoesäure ist ein Beispiel für die Chlorobenzoesäuren.

Nach dem Verfahren gemäß der vorliegenden Erfindung wird die Reaktion der Verbindung der Formel (2) mit Wasserstoffperoxid in Gegenwart der im Vorstehenden beispielhaft genannten organischen Carbonsäure in dem im Vorstehenden beispielhaft genannten aprotischen Lösungsmittel unter durch Schwefelsäure herbeigeführten sauren Bedingungen durchgeführt. Die Menge der verwenderten organischen Carbonsäure kann so klein sein, daß sie nicht mehr als 1 mol bezogen auf 1 mol der

Verbindung der Formel (2) beträgt. Beispielsweise beträgt die Menge der organischen Carbonsäure 0,05 bis 1 mol, vorzugsweise etwa 0,1 bis etwa 0,8 mol pro 1 mol der Verbindung der Formel (2). Die Menge der Schwefelsäure kann klein sein. Beispielsweise beträgt ihre Menge etwa 0,01 bis etwa 0,6 mol, vorzugsweise etwa 0,04 bis etwa 0,5 mol pro 1 mol der Verbindung der Formel (2).

Die Reaktion verläuft glatt bei Raumtemperatur. Beispielsweise liegt die angewandte Reaktionstemperatur bei etwa 0 °C bis etwa 25 °C, vorzugsweise bei etwa 0 °C bis etwa 20 °C. Die Reaktionszeit kann entsprechend gewählt werden und beträgt beispielsweise etwa 2 Stunden bis etwa 4 Stunden.

Nach dem Verfahren gemäß der vorliegenden Erfindung kann die Verbindung der Formel (1), die eine hohe Reinheit aufweist, nach der Beendigung der Reaktion leicht auf die folgende Weise aus der Reaktionsmischung abgetrennt und isoliert werden.

Da bei der Reaktion das aprotische organische Lösungsmittel verwendet wird, trennt sich die Reaktionsmischung in eine das angestrebte Produkt enthaltende organische Schicht und eine wäßrige Schicht. Die wäßrige Schicht wird entfernt, und die organische Schicht wird mit einem Alkali behandelt. Die Mischung wird mit Wasser gewaschen, und dann wird das Lösungsmittel abdestilliert, wodurch das gewünschte Produkt erhalten wird. Je nach den Erfordernissen kann die Reinheit des gewünschten Produkts durch Umkristallisation oder Destillation weiter gesteigert werden. Da die abgetrennte wäßrige Schicht einen sehr niedrigen CSB-Wert und eine sehr niedrige Gesamt-P-Konzentration in den Fällen, in denen die Verbindung der Formel (2) organisch gebundenen Phosphor enthält, aufweist, kann sie in einem einfachen Arbeitsgang abgeleitet werden, beispielsweise nach Behandlung mit unterchloriger Säure.

Die mit Hilfe des Verfahrens der vorliegenden Erfindung erhaltenen Verbindungen sind wertvolle chemische Verbindungen, die als Pestizide (Herbizide, Insektizide, Fungizide) oder als Zwischenprodukte für die Herstellung einer breiten Mannigfaltigkeit von Verbindungen auf dem Gebiet der organischen Chemie (z. B. auf den Gebieten der Pharmazeutika, der Pestizide, der Farbstoffe etc.) verwendet werden können.

Die vorliegende Erfindung wird durch die folgenden Synthesebeispiele und Vergleichs-Synthesebeispiele näher erläutert.

Synthesebeispiel 1 (Verfahren der vorliegenden Erfindung)

1,46 kg (10 mol) Di-n-butylthioether der Formel

$$n-C_4H_9-S-H_9C_4-n$$

wurden in 1,5 l Toluol gelöst, und 0,4 l Ameisensäure und 0,4 l 50-proz. Schwefelsäure wurden hinzugefügt. Die Mischung wurde mit Eis gekühlt, und unter Rühren wurden 1,07 kg 35-proz. Wasserstoffperoxid (11 mol) tropfenweise zugesetzt, wobei die Temperatur auf Raumtemperatur oder darunter gehalten wurde. Nach der Zugabe wurde die Mischung zur Vervollständigung der Umsetzung 4 h bei Raumtemperatur gerührt. Die wäßrige Schicht wurde von der Reaktionsmischung abgetrennt. Die organische Schicht wurde mit einer 20-proz. wäßrigen Natriumhydroxid-Lösung neutralisiert, und die wäßrige Schicht wurde abgetrennt. Die organische Schicht wurde mit Wasser gewaschen, und das Toluol wurde durch Destillation unter vermindertem Druck entfernt, wonach 1,46 kg (Ausbeute 90 %) Di-n-butylsulfoxid als angestrebtes Produkt erhalten wurden. Der Schmelzpunkt des Produkts lag bei 31 °C bis 32 °C. Gaschromatographische und flüssigkeitschromatographische Analyse ergaben eine Reinheit des Produkts von 96,5 % und eine Gesamt-Ausbeute von 86,9 %. Di-n-butylsulfon als Nebenprodukt wurde in einer Menge von weniger als 1 % gebildet.

Synthesebeispiel 2

Nach der gleichen Arbeitsweise wie in Synthesebeispiel 1 wurden bei Einsatz von 2,575 kg (10 mol) 4-Chlorobenzyl-N,N-diethylthiolcarbamat der nachstehenden Formel

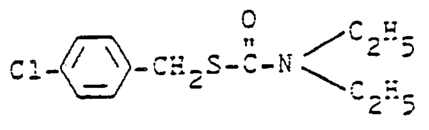

2,6 kg (Ausbeute 95 %) der angestrebten Verbindung der nachstehenden Formel

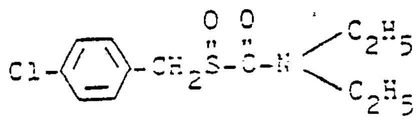

erhalten. $n_D^{20}$ 1,562 9 ; Reinheit 95 % ; Gesamt-Ausbeute 90,25 %.

Vergleichssynthesebeispiel 1

257,5 g (1 mol) 4-Chlorobenzyl-N,N-diethylthiolcarbamat der Formel

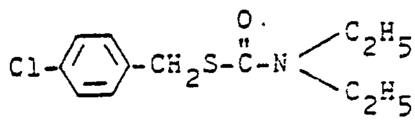

wurden rasch zu einer Lösung von 24,6 g (1,2 mol) m-Chloroperoxybenzoesäure in 4 l Chloroform hinzugegeben, die in einem Eisbad auf 3 °C gekühlt war. Zu dieser Zeit stieg die Temperatur der Reaktionsmischung auf 15 °C an. Dieser Zustand wurde 2 h aufrechterhalten, und die Reaktionsmischung wurde filtriert. Das Chloroform wurde aus dem Filtrat abdestilliert, wonach 241 g (Ausbeute 88 %) des angestrebten Produkts der nachstehenden Formel

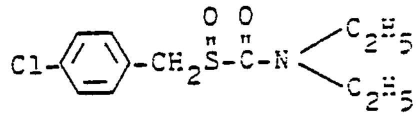

erhalten wurden. Reinheit 90 % ; Gesamt-Ausbeute 79,2 %.

Nach den gleichen Arbeitsweisen wie im Vorstehenden angegeben und unter den in der Tabelle 1 aufgeführten Bedingunden wurden die in der Tabelle 1 dargestellten Produkte erhalten.

(Siehe Tabelle 1 Seite 7 ff.)

Tabelle 1

| Beispiel | | Synthesebeispiel 3 | Vergleichs-synthesebeispiel 2 |
|---|---|---|---|
| Reaktand | | $(C_2H_5O)_2\overset{S}{\overset{\|}{P}}-SC_2H_4SC_2H_5$ | $(C_2H_5O)_2\overset{S}{\overset{\|}{P}}-SC_2H_4SC_2H_5$ |
| Menge | kg | 4,12 | 4,12 |
| | mol | 15 | 15 |
| Produkt | | $(C_2H_5O)_2\overset{S}{\overset{\|}{P}}-SC_2H_4\overset{O}{\overset{\|}{S}}C_2H_5$ | $(C_2H_5O)_2\overset{S}{\overset{\|}{P}}-SC_2H_4\overset{O}{\overset{\|}{S}}C_2H_5$ |
| Menge | kg | 4,25 | 3,94 |
| Lösungsmittel | | Toluol (4 l) | Methanol (5 l) |
| Säuren | | Ameisensäure (0,6 l)<br>50-proz.Schwefelsäure (0,6 l) | 50-proz.Schwefelsäure (0,04 l) |
| $H_2O_2$ (35 %) | kg | 1,65 | 1,65 |
| | mol | 17 | 17 |
| Reaktions- | | | |
| temperatur | | 0-10°C | Raumtemperatur |
| zeit | | 4 h | 4 h |
| Ausbeute | % | 97,0 | 90,4 |
| Reinheit | % | 95,0 | 59,4 |
| Gesamt-Ausbeute | % | 92,2 | 53,7 |
| Eigenschaften | | $n_D^{20}$ 1,5370 | |

Tabelle 1 (Fortsetzung)

| Beispiel | | Synthesebeispiel 4 | Synthesebeispiel 5 |
|---|---|---|---|
| Reaktand | | 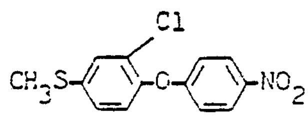 | 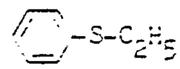 |
| Menge | kg | 2,96 | 1,38 |
| | mol | 10 | 10 |
| Produkt | | 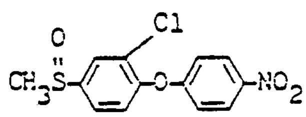 | 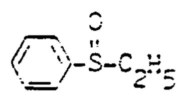 |
| Menge | kg | 2,96 | 1,47 |
| Lösungsmittel | | Chloroform (3 l) | Toluol (3 l) |
| Säuren | | Ameisensäure (0,4 l)<br>50-proz. Schwefelsäure (0,4 l) | Essigsäure (0,6 l)<br>50-proz. Schwefelsäure (0,6 l) |
| $H_2O_2$ (35 %) | kg | 1,07 kg | 1,07 kg |
| | mol | 11 | 11 |
| Reaktions- temperatur | | 0-10°C, | 0-10°C |
| zeit | | 4 h | 3 h |
| Ausbeute | % | 95,0 | 95,0 |
| Reinheit | % | 90,0 | 93,0 |
| Gesamt- Ausbeute | % | 85,5 | 88,4 |
| Eigenschaften | | Schmp. 93-94°C | $n_D^{20}$ 1,5583 |

Tabelle 1 (Fortsetzung)

| Beispiel | Synthesebeispiel 6 | Synthesebeispiel 7 |
|---|---|---|
| Reaktand | 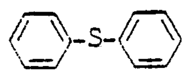 | 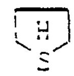 |
| Menge g | 186,3 | 88,16 |
| mol | 1 | 1 |
| Produkt | 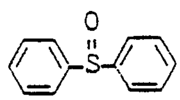 | 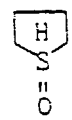 |
| Menge g | 202,3 | 98,9 |
| Lösungsmittel | Methylisobutylketon (0,3 l) | Toluol (0,3 l) |
| Säuren | Trichloroessigsäure (20 g)<br>50-proz. Schwefelsäure<br>(20 ml) | Ameisensäure (10 ml)<br>50-proz. Schwefelsäure<br>(20 ml) |
| $H_2O_2$ (35 %) g | 107 | 107 |
| mol | 1,1 | 1,1 |
| Reaktions- | | |
| temperatur | 0-10°C | 0-10°C |
| zeit | 4 h | 3 h |
| Ausbeute % | 100 | 95,0 |
| Reinheit % | 90,0 | 95,0 |
| Gesamt- | | |
| Ausbeute % | 90,0 | 90,25 |
| Eigenschaften | Schmp. 70°C | Sdp.106-107°C/20 mbar<br>(15 mmHg) |

Tabelle 1 (Fortsetzung)

| Beispiel | | Vergleichs-synthesebeispiel 3 |
|---|---|---|
| Reaktand | | 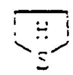 |
| Menge | g | 88,16 |
| | mol | 1 |
| Produkt | | 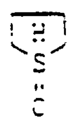 |
| Menge | g | 88,5 |
| Lösungsmittel | | Aceton (250 ml) |
| Säuren | | |
| $H_2O_2$ (35 %) | g | 107 |
| | mol | 1,1 |
| Reaktions-temperatur | | Raumtemperatur |
| Reaktions-zeit | | 5 h |
| Ausbeute | % | 85,0 |
| Reinheit | % | 85,0 |
| Gesamt-Ausbeute | % | 72,25 |
| Eigenschaften | | |

**Patentansprüche**

1. Verfahren zur Herstellung eines Sulfoxids der nachstehenden Formel

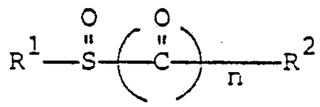 (1)

in der $R^1$ und $R^2$ gleich oder verschieden sind und jeweils für eine Alkyl-, Alkenyl- oder Alkinyl-Gruppe, die jeweils durch Halogen, Alkoxy, einen Phosphorsäureester-Rest, eine Phenyl-Gruppe oder im Falle von Alkyl eine durch 1 bis 5 Halogenatome oder 1 bis 5 Cyano-, Nitro-, Alkyl-, Alkoxy- oder Phenyloxy-Gruppen substituierte Phenyl-Gruppe substituiert sein können, eine Aryl-Gruppe, die durch ein Halogen-Atom oder eine Nitro-, Alkyl-, Alkoxy-, Cyano-, Alkenyl-, Alkinyl-, Aryloxy-, Alkylcarbonyl- oder Alkoxycarbonyl-Gruppe substituiert sein kann, oder eine Gruppe der Formel

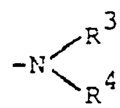

stehen, in der

$R^3$ und $R^4$ gleich oder verschieden sind und jeweils für ein Wasserstoff-Atom oder eine Alkyl-, Alkenyl-, Alkinyl- oder Phenyl-Gruppe stehen oder $R^3$ und $R^4$ miteinander verbunden sein können und einen $C_3$- bis $C_6$-Ring bilden, den gegebenenfalls ein anderes Hetero-Atom enthält, und

n 0 oder 1 ist, und wenn n 0 ist, $R^1$ und $R^2$ miteinander verbunden sein können, so daß sie einen Ring mit $C_4$ bis $C_6$ bilden,

das die Reaktion eines Thioethers oder eines Thiolcarbonsäure-Derivats der Formel

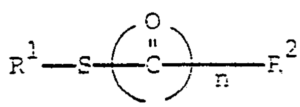 (2)

in der $R^1$, $R^2$ und n die im Vorstehenden angegebenen Bedeutungen haben, zur Oxidation desselben mit Wasserstoffperoxid umfaßt,

dadurch gekennzeichnet, daß die Reaktion in einem aprotischen Lösungsmittel in Gegenwart einer organischen Carbonsäure unter durch Schwefelsäure herbeigeführten sauren Bedingungen durchgeführt wird.

2. Verfahren nach Anspruch 1, dadurch gekennzeichnet, daß die organische Carbonsäure ausgewählt ist aus der aus Ameisensäure, Essigsäure, Propionsäure und halogensubstituierten Produkten dieser Säuren bestehenden Gruppe.

3. Verfahren nach Anspruch 1, dadurch gekennzeichnet, daß die Menge der organischen Carbonsäure 0,1 bis 0,8 mol pro 1 mol des Thioethers oder Thiolcarbonsäure-Derivats beträgt.

4. Verfahren nach Anspruch 1, dadurch gekennzeichnet, daß das aprotische Lösungsmittel mindestens eines aus der aus Kohlenwasserstoffen, halogenierten Kohlenwasserstoffen, Ethern und Ketonen bestehenden Gruppe ist.

**Claims**

1. Process for the preparation of a sulphoxide of the following formula

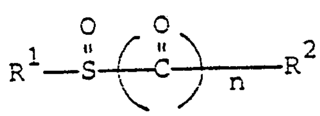 (1)

in which $R^1$ and $R^2$ are identical or different and each represent an alkyl, alkenyl or alkinyl group, each of which can be substituted by halogen, alkoxy, a phosphoric acid ester radical, a phenyl group or, in the case of alkyl, a phenyl group substituted by 1 to 5 halogen atoms or 1 to 5 cyano, nitro, alkyl, alkoxy or phenyloxy groups, an aryl group which can be substituted by a halogen atom or a nitro, alkyl, alkoxy, cyano, alkenyl, alkinyl, aryloxy, alkylcarbonyl or alkoxycarbonyl group, or a group of the formula

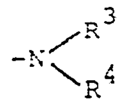

in which

$R^3$ and $R^4$ are identical or different and each represent a hydrogen atom or an alkyl, alkenyl, alkinyl or phenyl group or $R^3$ and $R^4$ can be bonded to each other and form a $C_3$ to $C_6$ ring which optionally contains another hetero atom and

n is 0 or 1, and when n is 0, $R^1$ and $R^2$ can be bonded to each other so that they form a ring with $C_4$ to $C_6$,

which comprises reacting a thioether or a thiolcarboxylic acid derivative of the formula

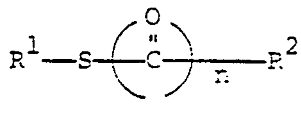

(2)

in which $R^1$, $R^2$ and n have the meanings given above, for the oxidation thereof, with hydrogen peroxide, characterised in that the reaction is carried out in an aprotic solvent in the presence of an organic carboxylic acid under acid conditions produced by means of sulphuric acid.

2. Process according to Claim 1, characterised in that the organic carboxylic acid is selected from the group consisting of formic acid, acetic acid, propionic acid and halogen-substituted products of these acids.

3. Process according to Claim 1, characterised in that the quantity of the organic carboxylic acid is 0.1 to 0.8 mol per 1 mol of the thioether or thiolcarboxylic acid derivative.

4. Process according to Claim 1, characterised in that the aprotic solvent is at least one from the group consisting of hydrocarbons, halogenated hydrocarbons, ethers and ketones.

**Revendications**

1. Procédé de préparation d'un sulfoxyde de formule

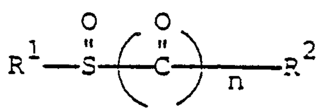

(1)

dans laquelle $R^1$ et $R^2$, ayant des significations identiques ou différentes, représentent chacun un groupe alkyle, alcényle ou alcynyle qui peuvent chacun être substitués par des halogènes, des groupes alcoxy, un reste d'ester phosphorique, un groupe phényle ou bien, dans le cas du groupe alkyle, un groupe phényle substitué par 1 à 5 atomes d'halogènes ou 1 à 5 groupes cyano, nitro, alkyle, alcoxy ou phényloxy, un groupe aryle qui peut être substitué par un atome d'halogène ou un groupe nitro, alkyle, alcoxy, cyano, alcényle, alcynyle, aryloxy, alkylcarbonyle ou alcoxycarbonyle, ou bien un groupe de formule

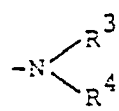

dans laquelle

$R^3$ et $R^4$, ayant des significations identiques ou différentes, représentent chacun un atome d'hydrogène ou un groupe alkyle, alcényle, alcynyle ou phényle ou bien $R^3$ et $R^4$ sont reliés ensemble et forment un cycle en $C_3$ à $C_6$ qui contient, le cas échéant, un autre hétéroatome, et

n est égal à 0 ou 1 et, lorsque n est égal à 0, $R^1$ et $R^2$ peuvent être reliés ensemble en formant un cycle en $C_4$ à $C_6$, par oxydation d'un thioéther ou d'un dérivé d'acide thio-carboxylique de formule

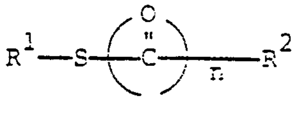

(2)

dans laquelle $R^1$, $R^2$ et n ont les significations indiquées ci-dessus, par réaction avec du peroxyde d'hydrogène,

caractérisé en ce que la réaction est effectuée dans un solvant aprotonique en présence d'un acide organique carboxylique dans un milieu acide créé par l'acide sulfurique.

2. Procédé selon la revendication 1, caractérisé en ce que l'acide organique carboxylique est choisi dans le groupe consistant en l'acide formique, l'acide acétique, l'acide propionique et les dérivés halogénés de ces acides.

3. Procédé selon la revendication 1, caractérisé en ce que la quantité de l'acide organique carboxylique représente de 0,1 à 0,8 mol par mol du thioéther ou du dérivé d'acide thiol-carboxylique.

4. Procédé selon la revendication 1, caractérisé en ce que le solvant aprotonique consiste en au moins un solvant du groupe des hydrocarbures, des hydrocarbures halogénés, des éthers et des cétones.